# EUROPEAN PATENT APPLICATION

(11) **EP 4 844 379 A1**
(43) Date of publication of application: **25.11.2026**
(21) Application number: 25207560.1
(22) Date of filing: 08.10.2025
(51) Int. Cl.: G01N 21/45, G01N 21/77, G01K 1/00, G01N 33/38

(54) **SYSTEM AND METHOD FOR FIBER OPTICAL RELATIVE HUMIDITY AND TEMPERATURE SENSING, AND USES THEREOF**

(30) Priority: 23.05.2025 PT 2025120286
(71) Applicant: INESC TEC - Instituto de Engenharia de Sistemas e Computadores, Tecnologia e Ciência, 4200-465 Porto (PT)
(72) Inventor: PEREIRA DE FARIA, RITA, 4200-465 PORTO (PT); SAMPAIO MENDES, JOÃO PEDRO, 4200-465 PORTO (PT); MARQUES MARTINS DE ALMEIDA, JOSÉ MANUEL, 4200-465 PORTO (PT); COSTA COELHO, LUIS CARLOS, 4200-465 PORTO (PT)
(74) Representative: Patentree

(57) **Abstract**

The disclosure relates to an optical sensing device for temperature and relative humidity dual-measurement of a cementitious material, comprising an optical fiber for receiving and returning an optical signal for transducing the measured temperature and relative humidity; a hydrophilic polymer layer deposited onto a tip of the optical fiber forming a Fabry-Perot interference, FPI, optical cavity for humidity measurement; a Fiber Bragg Grating, FBG, inscribed in the optical fiber for temperature measurement; and a chamber comprising the tip of the optical fiber; wherein said chamber is enclosed by a vapor-permeable, liquid-impermeable sheet.

## Description

### TECHNICAL FIELD

The present disclosure relates to the field of monitoring concrete conditions and structural health, and more specifically to optical sensors for simultaneous temperature and relative humidity (RH) measurement, namely during the curing of concrete structures, particularly those designed for harsh environments such as offshore wind turbine foundations.

### BACKGROUND

Monitoring temperature and relative humidity (RH) levels during the curing of concrete structures is essential for ensuring quality, strength, and durability. Temperature influences the rate of concrete hydration, which directly affects its strength. Excessive moisture can cause issues such as steel reinforcement corrosion, alkali-silica reaction, and degradation of concrete strength, while insufficient moisture may result in reduced strength and increased susceptibility to cracking [1]. Precise monitoring of temperature and RH optimizes the curing process, minimizes structural damage risks, and extends the concrete's service life. However, traditional capacitive or resistive temperature and RH sensors often face challenges like drift, hysteresis, and performance degradation in harsh environments. Optical sensors offer a promising alternative to address these limitations [2].

One widely recognized optical solution for temperature sensing is the Fiber Bragg Grating (FBG), which consists of an optical fiber with periodic spatial modulation of the core's refractive index. This configuration selectively back-reflects light within a narrow spectral range centered at the Bragg wavelength, *λ_{B}*, which is defined by the equation: *λ_{B} =* 2*nₑ*Λ (1).

Here, *nₑ* corresponds to the fiber core's effective refractive index (ERI), and A to the grating period. The FBG operates as a temperature sensor due to the temperature-induced variations in the grating period and ERI, leading to a corresponding shift in the Bragg wavelength.

RH sensing is usually a more complex challenge than temperature sensing due to the broader range of sensing mechanisms and materials.

These facts are disclosed in order to illustrate the technical problem addressed by the present disclosure.

### GENERAL DESCRIPTION

The present disclosure relates to the field of condition monitoring and structural health monitoring, and more specifically to optical sensors for simultaneous temperature and relative humidity (RH) measurement, namely during the curing of concrete structures, particularly those designed for harsh environments such as offshore wind turbine foundations.

Concrete structures require accurate and continuous precise monitoring of temperature and humidity during curing to ensure optimal mechanical strength and prevent defects like cracking, internal stress buildup, or shrinkage. To address these requirements, a compact optical sensing system was developed using a single mode optical fiber that can be embedded directly within the concrete structure. The system functions simultaneously as a temperature and humidity sensor when paired with a spectral interrogation unit operating in the 1500-1600 nm range. Temperature monitoring is achieved through an FBG, while humidity sensing is facilitated by a Fabry-Perot Interferometer (FPI) at the fiber tip. The interferometer cavity is formed with a layer of hydrophilic polymer such as polyvinylpyrrolidone (PVP). Initial air humidity sensor tests showed a significant change in the interference period with RH, demonstrating low hysteresis and high reproducibility. A sensor calibration test revealed an approximately 3 nm reduction in the interference period when RH increased from 55% to 95%, with results suggesting a quadratic relationship between the interference period and RH values.

The presented configuration uses a Fabry-Perot (FP) interferometer on a single-mode fiber tip (SMF) made with a hydrophilic polymer layer for simplicity and effectiveness. The polymer layer absorbs and expels water as surrounding RH changes, modifying its ERI and cavity thickness. For a low-finesse, single-layer FP interferometer, the interference pattern frequency observed in the reflection spectra is described by the equation: $ω = \frac{2 π}{T} = 4 {πn}_{pol} L$ where *nₚₒₗ* is the ERI of the polymer, and L represents the cavity's length. However, both *nₚₒₗ* and L change simultaneously, and depending on the polymer's characteristics, one may increase while the other decreases. Given the complexity of precisely modelling these changes, the most practical approach is to establish a calibration curve for each sensor, mapping the relationship between the interference period and RH levels.

The goal is to create a compact, reliable optical sensing system for temperature and RH monitoring during concrete curing, specifically designed for offshore wind turbine construction, but not limited to. The sensors also support continuous monitoring post-installation, meeting the rigorous endurance requirements imposed by offshore operations.

The present disclosure includes a compact dual-parameter optical sensor that integrates a FBG and a FPI within a single fiber structure for simultaneous monitoring of temperature and RH during concrete curing. The solution with a hydrophilic polymer such as Polydimethylsiloxane, PDMS, forms a vapor-permeable but water-resistant membrane that enables reliable long-term sensing even in harsh, high-humidity environments such as offshore concrete structures.

In a preferred embodiment, the FPI is constructed at the tip of a ceramic ferrule, onto which a thin film of titanium dioxide (TiO₂) is first deposited, followed by a hydrophilic polymer layer. This configuration creates a low-finesse interferometric cavity for RH sensing. The TiO₂ layer serves a dual purpose: it ensures sufficient refractive index contrast to maintain optical interference over the full humidity range, and increases the reflected optical power.

The polymer layer, preferably composed of PVP, responds to changes in ambient humidity by absorbing or desorbing water, thereby modifying its ERI and thickness. The structure protection is further designed for a dual effect: the polymer cavity is accessible to vapor diffusion but shielded from bulk water, preserving sensor calibration and performance during immersion or high-humidity curing environments such as fresh concrete.

The present disclosure relates to an optical sensing device for temperature and relative humidity dual-measurement of a cementitious material, comprising:
an optical fiber for receiving and returning an optical signal for transducing the measured temperature and relative humidity;
a hydrophilic polymer layer deposited onto a tip of the optical fiber forming a Fabry-Perot interference, i.e. a Fabry-Perot Interferometer, FPI, optical cavity for humidity measurement;
a Fiber Bragg Grating, FBG, inscribed in the optical fiber for temperature measurement; and
a chamber comprising the tip of the optical fiber;
wherein said chamber is enclosed by a vapor-permeable, liquid-impermeable barrier, in particular a vapor-permeable, liquid-impermeable sheet. The disclosed structure is designed to allow water vapor to pass through the barrier and interact with the polymer layer while minimizing or preventing the ingress of liquid water, thereby forming an arrangement that ensures sensor functionality even in near-saturated or submerged environments. The sheet is a particularly advantageous format for this barrier.

In an embodiment, the device comprises a metal oxide layer between the hydrophilic polymer layer and the tip of the optical fiber.

In an embodiment, the metal oxide layer is a Titanium Dioxide, TiO2, layer.

In an embodiment, the vapor-permeable, liquid-impermeable sheet is a membrane, in particular a Polydimethylsiloxane, PDMS, membrane.

In an embodiment, the hydrophilic polymer layer is selected from a group consisting of polyvinylpyrrolidone, polyimide, hydroxyethyl cellulose, and polyethylene glycol, preferably polyvinylpyrrolidone, PVP.

In an embodiment, the optical fiber comprises a FBG optical fiber section spliced to a FPI optical fiber section.

In an embodiment, the optical fiber is coupled to a fiber optic connector.

In an embodiment, the device comprises a ceramic ferrule wherein the tip of the optical fiber is inserted into the ceramic ferrule, i.e. the ceramic ferrule receives the tip of the optical fiber in an opening which extends to a surface of the ceramic ferrule such that the tip of the optical fiber is flush with said surface of the ceramic ferrule, to provide a base surface for deposition of the hydrophilic polymer layer.

In an embodiment, the optical fiber is a single-mode fiber operating in the infrared region, preferably between 1500 nm and 1600 nm.

In an embodiment, the device comprises an electronic data processor configured for determining relative humidity by measuring a change in an FPI interference pattern of the reflected optical signal. In an embodiment, the device comprises an electronic data processor further configured for determining temperature by measuring an FBG interference pattern of the reflected optical signal.

In an embodiment, the electronic data processor is configured for quantifying said change by measuring spectral separation between consecutive interference peaks or the mean spectral distance between such peaks.

In an embodiment, analysis of the reflected optical signal excludes the FBG reflection peak.

In an embodiment, the device comprises a protective encapsulation comprising a ferrule housing, stainless steel tube, and/or resin encapsulation.

The present disclosure also relates to a method of fabricating the optical sensing device, comprising:
optionally, applying a titanium dioxide, TiO₂, layer over the fiber tip;
optionally, arranging the tip of the optical fiber on a ceramic ferrule such that the tip of the optical fiber is flush with a surface of the ceramic ferrule;
dip-coating the tip of the optical fiber in a hydrophilic polymer, for example polyvinylpyrrolidone, PVP, for preparing a Fabry-Perot cavity on said tip;
integrating a Fiber Bragg Grating sensor on said optical fiber; and
optionally, assembling a protective housing comprising a ferrule and outer casing.

The present disclosure also relates to a method of calibrating the optical sensing device, comprising:
exposing the relative humidity sensor to controlled humidity environments and
recording the reflected spectra;
establishing a calibration curve correlating relative humidity values to free spectral range, FSR, measurements; and
correlating Bragg wavelength shifts to known temperature values for temperature calibration.

The present disclosure is conceived for the monitoring of the integrity of structures, namely concrete structures, but also has use cases for the agriculture, food industry, pharmaceutical industry and pyrotechnics.

Where elements are described as being connected or connectable, they may be directly connected. Where elements are described as being coupled or capable to be coupled, they may be linked by one or more intervening or interposing elements.

### BRIEF DESCRIPTION OF THE DRAWINGS

The following figures provide preferred embodiments for illustrating the disclosure and should not be seen as limiting the scope of invention.
**Figure 1****:** Diagram of the relative humidity sensor based on a polymer Fabry-Perot interferometer.
**Figure 2****:** Magnified photo of a completed RH sensor a polymer Fabry-Perot interferometer.
**Figure 3****:** Diagram of the sensing system protection setup.
**Figure 4****:** Photos of a) the sensing system with protection for the RH sensor and b) the complete protection setup, with the fiber inside the stainless-steel tube.
**Figure 5****:** RH calibration curve: a) FP optical response and FBG peak at 1590 nm; b) Calibration curve for RH variations of the produced sensing configuration, derived from four 55%-95%-55% RH step cycles.
**Figure 6****:** Sensor response in curing concrete: Timeline of an in-concrete test with highlighted timestamps when the sample was submerged in and removed from water.
**Figure 7****:** Sensing integration diagram.
**Figure 8****:** Simplified diagram of the optical sensing system with its two main components: a Fiber Bragg Grating on the left and a polymer Fabry-Perot interferometer on the right. The diagram is not to scale.
**Figure 9****:** Schematic structure of a Fiber Bragg Grating (FBG). The fiber's core has a periodic modulation of its refractive index with a period of *Λ*. The incident, transmitted and reflected spectra are numbered 1,2 and 3, respectively.
**Figure 10****:** Diagram of the resulting transmitted and reflected spectra for light passing through an FBG, given the initial incident spectrum.
**Figure 11****:** Schematic structure of a fiber Fabry-Perot Interferometer (FPI), where a different material is applied at the fiber tip. The incident and reflected spectra are numbered 1 and 2, respectively. The diagram is not to scale.
**Figure 12****:** Example of a reflection spectrum obtained with the current system. The interference pattern of the FPI is highlighted, with its peaks identified. The FBG peak is highlighted.

### DETAILED DESCRIPTION

The present disclosure relates to a compact optical sensing system for simultaneous monitoring of temperature and RH for cementitious materials, which is particularly useful during the curing of said cementitious materials, particularly concrete curing, particularly for harsh environments such as offshore environments, for example offshore wind turbine foundations.

The present disclosure is conceived for the monitoring of the integrity of structures, namely concrete structures, but also has use cases for the agriculture, food industry, pharmaceutical industry and pyrotechnics.

The present disclosure includes a compact optical sensing system that combines a FBG for temperature sensing and a low-finesse FPI for RH sensing, both implemented within a single optical fiber. The FPI is formed at the tip of a ceramic ferrule using a hydrophilic polymer layer, such as polyvinylpyrrolidone, deposited over a titanium dioxide intermediate layer to ensure consistent optical interference and maintain reflectivity across a wide humidity range. The solution allows vapor diffusion while preventing liquid water ingress, enabling reliable operation under saturated or submerged conditions. The sensing head is preferably enclosed in a robust protective housing, allowing for continuous, passive monitoring of environmental conditions via reflection spectrum analysis.

In an embodiment, the fabrication process comprises three main stages: i) fabrication of the FP cavity using a hydrophilic polymer; ii) implementation of an FBG on the FP fiber body for temperature monitoring; and iii) enclosing the structure to enhance robustness. The compact design of the sensing system allows seamless integration with an interrogation unit operating in the 1500-1600 nm range, enabling continuous environmental monitoring through reflection spectra analysis.

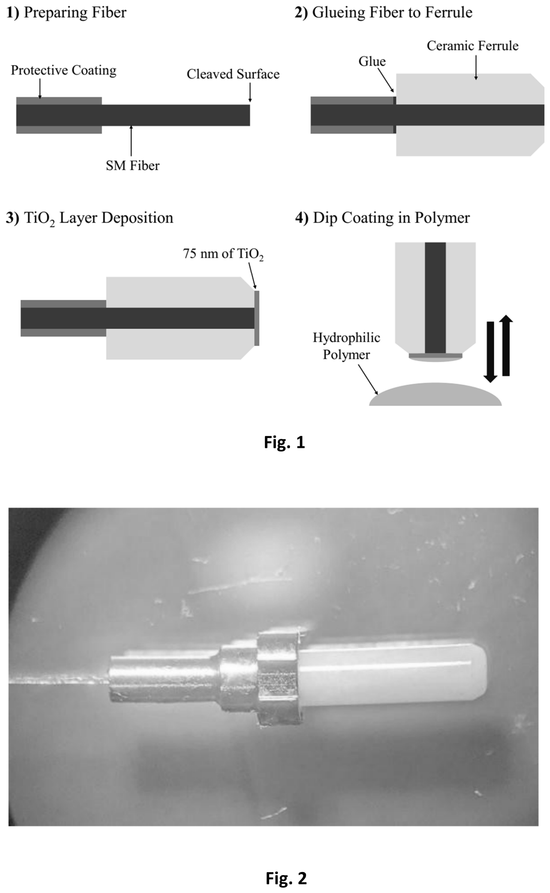

In an embodiment, the RH sensor fabrication process is summarized in **Figure 1****.** It begins by inserting a cleaved SMF tip (1) into a ceramic ferrule (2). The interferometer layers were built on a ceramic ferrule instead of directly on a bare fiber to facilitate the dip coating process. The ferrule provides a larger surface area for the polymer to adhere to, resulting in more consistent coating outcomes.

In an embodiment, subsequently, a 75 nm layer of TiO₂ was deposited onto the ferrule tip (3) using magnetron sputtering. This was followed by applying a polymer layer via dip coating (4) in a solution of 15% (wt/wt) PVP K90 in water. The PVP was selected from various hydrophilic polymers for its superior durability and repeatability over a humidity range from 50% to nearly 100% RH. Because its RI is higher than that of glass, there may be an RH range (depending on the desired RH range) where *nₚₒₗ* approaches that of glass, reducing the contrast at the polymer-glass interface. The TiO₂ layer addresses this issue by ensuring consistent reflection at the interface, maintaining the Fabry-Perot (FP) cavity across the entire desired RH range.

In an embodiment, during the dip-coating process, the reflection spectrum was monitored in real-time using an interrogation unit (HBK FiberSensing SI22 Braggmeter), dipping until the desired spectral properties were achieved. These properties can be then repeatedly obtained by using the same dipping conditions. **Figure 2** shows the completed RH sensor. Before use, the sensor is preferably conditioned in a high-humidity environment to stabilize the polymer layer. Finally, each sensor is preferably calibrated in a controlled humidity chamber to establish a correlation between the interference period and RH values.

In an embodiment, a prefabricated FBG with a Bragg wavelength between 1500 and 1600 nm was selected and spliced onto the RH sensor fiber. An FC-APC connector (ferrule connector - angled physical contact) was spliced onto the opposite end of the FBG fiber to facilitate monitoring.

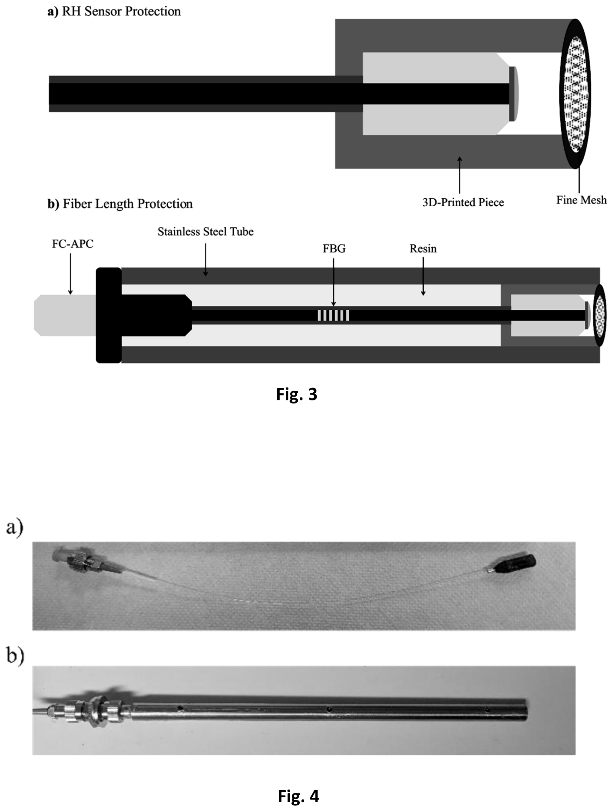

In an embodiment, the sensing system requires two types of protection: specialized protection for the RH sensor tip, which maintains vapor access while preventing harmful material intrusion, and robust shielding along the entire fiber length to safeguard it from environmental stresses. **Figures 3** and **4** show the protection setup, which uses a custom 3D-printed ferrule housing, a stainless-steel tube, and resin encapsulation for complete fiber protection.

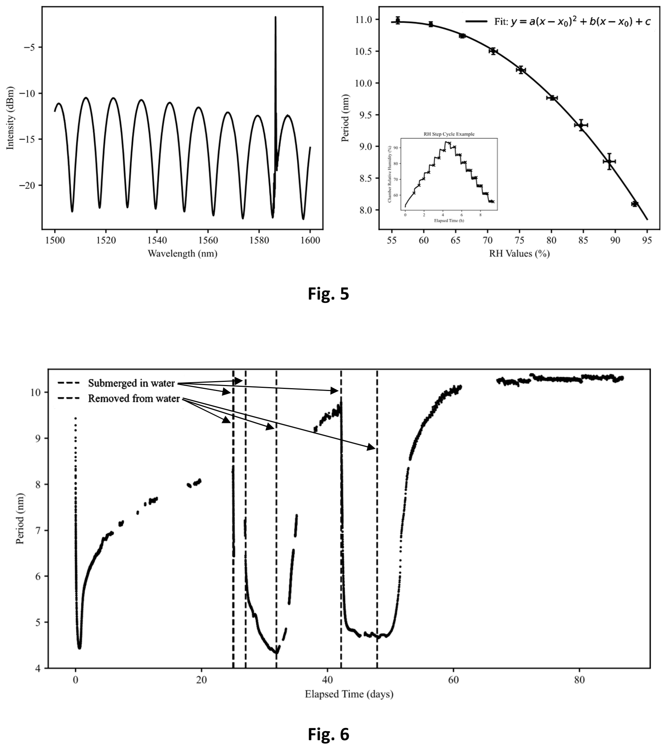

In an embodiment, **Figure 5** a) illustrates the reflection spectrum of one of the fabricated sensing configurations, highlighting the interference pattern of the FP cavity and the FBG reflection peak near 1590 nm. **Figure 5** b) displays the calibration data obtained in a controlled humidity chamber, where RH levels were incrementally stabilized over defined time intervals. Previous tests indicated a sensor response time of approximately 30 minutes, primarily influenced by the polymer's water absorption rate and the diffusion rate of water vapor through the mesh. Considering this response time, the sensor demonstrated excellent reproducibility during humidity chamber tests.

It is noted that, in an embodiment, a membrane may be a mesh.

However, testing in a humidity chamber does not replicate concrete conditions, where RH stays near 100% and liquid water is present for extended periods. As a result, testing within concrete was necessary. **Figure 6** presents the results of a preliminary test conducted within a curing concrete sample. The sample was repeatedly submerged and removed from water to observe the sensor's response. While the RH sensor consistently reacted, its calibration could become unreliable, likely due to water infiltration through the mesh. Enhanced protective solutions maybe then used, such as adding a silicone-based layer, like PDMS, to reinforce insulation of the mesh.

The present disclosure presents a compact optical sensing system that combines an FBG and a FP interferometer for temperature and RH monitoring during concrete curing, focusing on offshore wind turbine construction. Using PVP as a hydrophilic polymer for RH sensing via FP interferometry, the system demonstrated great reproducibility, low hysteresis, and a clear response to RH changes. Calibration results indicated a total period decrease of approximately 3 nm between 55% and 95% RH, with a general quadratic relation.

In the present disclosure, the system may comprise a metal tube to be embedded in concrete, with a single optical fiber exiting from the structure. The disclosure enables further advancements in protective systems for ensuring consistent and reliable performance under high-humidity conditions. The disclosed optical sensing system represents a step forward in continuous structural monitoring and optimizing concrete curing processes.

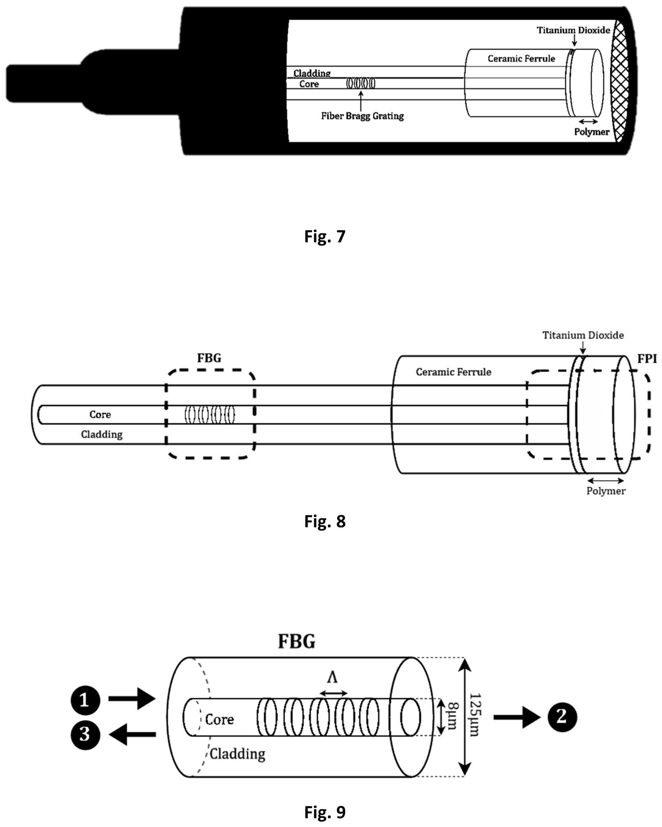

In an embodiment, it is also disclosed a diagram of the entire sensing setup and its protective measures. **Figure 8** presents a simplified version for clarity and ease of understanding.

In an embodiment, **Figure 7** presents a sensing setup diagram with the added protective structures.

In an embodiment, the setup utilizes single-mode fibers operating within the 1500 to 1600 nm wavelength range, which falls in the infrared spectrum. The system is meant to be analyzed through its reflection spectrum.

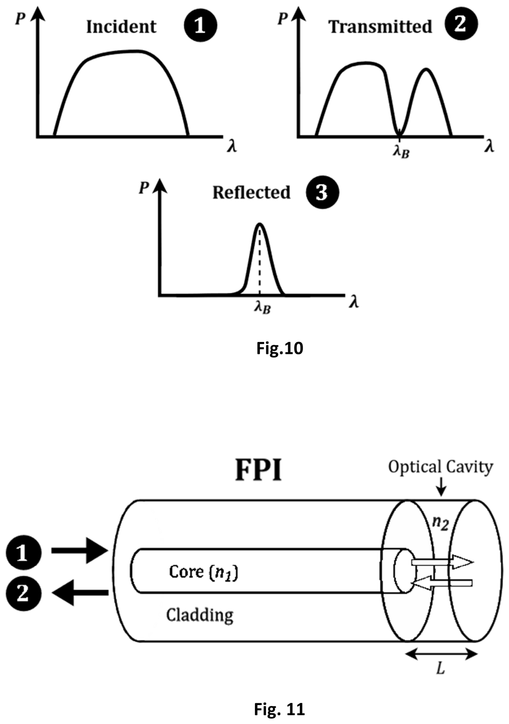

In an embodiment, first, as for the temperature sensor, a FBG is used. This structure comprises a fiber with a periodic spatial modulation of the core's refractive index (RI), with a period of *Λ,* as represented in **Figure 9****.** This configuration creates several periodic surfaces where light reflects, acting as partial mirrors. These surfaces originate successive reflection, inducing an interference phenomenon where a very narrow spectral range, centered at a certain Bragg wavelength, *λ_{B}*, is back-reflected. This results in an easily trackable peak on its reflection spectra, as shown in **Figure 10****.**

The position of the peak, *λ_{B}*, is given by the Bragg condition, Eq 1, where *Λ* is the fiber modulation period and *n_{eff}* is effective RI of light in the fiber. ${λ}_{B} = 2 {n}_{eff} Λ$ (1)

This configuration can be used as a temperature sensor by leveraging the natural thermal expansion and contraction of materials. When temperature rises, glass expands, *Λ* increases and the peak shifts to higher wavelengths (blue shift). Similarly, when temperature decreases, the glass contracts and both *Λ* and *λ_{B}* decrease (red-shift). Then, it is just a matter of calibrating T (° C) vs. *λ_{B}*(nm).

In an embodiment, the RH sensor operates as an FPI. It comprises an optical cavity formed by two partially reflective parallel mirrors, separated by a distance *L*. This configuration can be easily implemented in an optical fiber by introducing a different material at the fiber tip, which serves as the optical cavity, as illustrated in **Figure 11****.**

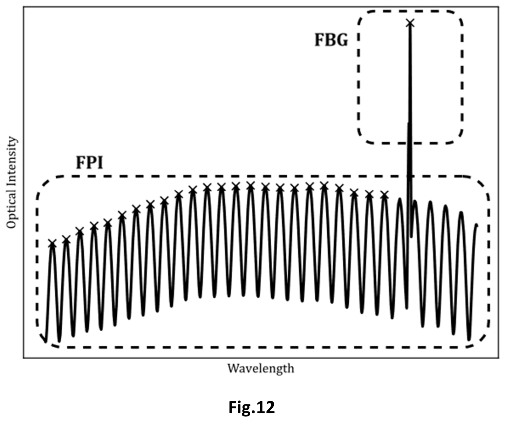

The RI contrast between the fiber core (*n*₁), the cavity material (*n*₂), and the surrounding environment (*n*₀) should be sufficiently high to ensure significant Fresnel reflection at both interfaces. This enables partial confinement of light within the cavity, leading to interference. The resulting intensity interference pattern (as seen in **Figure 12**) can be approximated by the superposition of the incident (*I*₁) and reflected (*I*₂) beams: $I \left(λ\right) = {I}_{1} \left(λ\right) + {I}_{2} \left(λ\right) + \sqrt{{I}_{1} \left(λ\right) {I}_{2} \left(λ\right)} cos \left(ϕ+{φ}_{0}\right)$ (2)
where *φ*₀ is the initial phase, and *ϕ* represents the phase difference between the beams due to the optical path difference, given by: $ϕ = \frac{4 {πn}_{2} L}{λ}$ (3)

This equation shows that the interference pattern shifts when either *n*₂ or *L* changes. To utilize this configuration as a humidity sensor, a hydrophilic polymer is used for the optical cavity. This polymer absorbs or releases water depending on the ambient relative humidity, leading to variations in both *n*₂ and *L*, thereby modifying the interference pattern.

Another approach to tracking these variations is by analyzing the Free Spectral Range (FSR), Δ*λ*, which represents the spectral separation between consecutive interference peaks: $Δ λ = \frac{{λ}^{2}}{2 {n}_{2} L}$ (4)
although Δ*λ* varies with wavelength, by having a sufficient number of visible peaks and averaging Δ*λ* all over the spectrum, the contribution of *n*₂*L* can essentially be isolated. Similar to FBGs, the sensor can then be calibrated by establishing the relation between RH (%) and Δ̅*̅λ̅*̅ (nm).

As previously mentioned, one of the factors that may limit the sensor's working range is the refractive index contrast. If the polymer reaches a point where *n*₂ ≈ *n*₁, the fiber-polymer interface will no longer produce significant Fresnel reflection, thereby compromising optical interference. To mitigate this issue, an intermediate material is often introduced between the polymer and the fiber to maintain sufficient contrast, even when *n*₂ ≈ *n*₁. In this system, a thin film of titanium dioxide is applied as an additional layer, as observed in **Figure 1****,** ensuring the interface retains its partial mirror behavior and preserves interference effects.

In an embodiment, to integrate both sensors into a single system, as illustrated in **Figure 1****,** the two fibers are simply fused together. This allows both interference phenomenon to coexist, resulting in a combined reflection spectrum as the one in **Figure 12****.**

To obtain temperature and RH measurements, the process can be reduced to basic peak detection. For the FBG sensor, the key step is identifying the peak wavelength *λ_{B}* and comparing it with the calibration curve T(°C) vs. *λ_{B}*(nm).

For the FPI sensor, the approach involves tracking all peaks that do not correspond to the FBG peak-typically, it is easier to focus on the peaks on one side of the FBG. The mean spectral distance between these peaks, Δ̅*̅λ̅*̅, is then calculated. Once again, the measurement is obtained by comparing Δ̅*̅λ̅*̅ with the calibration curve RH (%) vs Δ̅*̅λ̅*̅ (nm).

The present disclosure is financed by Component 5 - Capitalization and Business Innovation, integrated in the Resilience Dimension of the Recovery and Resilience Plan within the scope of the Recovery and Resilience Mechanism (MRR) of the European Union (EU), framed in the Next Generation EU, for the period 2021 - 2026, within project ATE, with reference 56.

The term "comprising" whenever used in this document is intended to indicate the presence of stated features, integers, steps, components, but not to preclude the presence or addition of one or more other features, integers, steps, components or groups thereof.

As used in the specification and claims, the singular forms "a", "an" and "the" include plural references unless the context clearly dictates otherwise. For example, the term "a sample" includes a plurality of samples, including mixtures thereof.

Notably, the figures and examples above are not meant to limit the scope of the present disclosure to a single implementation, as other implementations are possible by way of interchange of some or all the described or illustrated elements. Moreover, where certain elements of the present disclosure can be partially or fully implemented using known components, only those portions of such known components that are necessary for an understanding of the present disclosure are described, and detailed descriptions of other portions of such known components are omitted so as not to obscure the disclosure. In the present specification, an implementation showing a singular component should not necessarily be limited to other implementations including a plurality of the same component, and vice-versa, unless explicitly stated otherwise herein. Moreover, applicants do not intend for any term in the specification or claims to be ascribed an uncommon or special meaning unless explicitly set forth as such. Further, the present disclosure encompasses present and future known equivalents to the known components referred to herein by way of illustration.

The subject matter described above is provided by way of illustration only and should not be construed as limiting. Various modifications and changes can be made to the subject matter described herein without following the example embodiments and applications illustrated and described, and without departing from the true spirit and scope of the invention encompassed by the present disclosure, which is defined by the set of recitations in the following claims and by structures and functions or steps which are equivalent to these recitations.

The disclosure should not be seen in any way restricted to the embodiments described and a person with ordinary skill in the art will foresee many possibilities to modifications thereof.

The above-described embodiments are combinable.

The following claims further set out particular embodiments of the disclosure.

### References

[01] Hobbs DW, "Concrete deterioration: causes, diagnosis, and minimizing risk," Int Mater Rev 46(3): 117-144 (2001); doi:10.1179/095066001101528420
[02] Leung CKY, "Fiber optic sensors in concrete: the future," Ndt E Int 34(2):85-94 (2001); doi:10.1016/509638695(00)00033-5
[03] Su, D.; Qiao, X.; Rong, Q.; Sun, H.; Zhang, J.; Bai, Z.; Du, Y.; Feng, D.; Wang, Y.; Hu, M. and Feng, Z. "A fiber Fabry-Perot interferometer based on a PVA coating for humidity measurement", Optics Communication, Elsevier BV (2013), 311, 107-110
[04] Bernardo Dias, João Pedro Sampaio Mendes, José Manuel Marques Martins de Almeida and Luís Carlos Costa Coelho, "Simple optical fiber interferometer for dynamic measurement of refractive index and thickness of polymer films". IEEE Sensors Journal, 22(12):11732-11739 (6/2022); doi: 10.1109/jsen.2022.3171046

## Claims

1. An optical sensing device for temperature and relative humidity dual-measurement of a material or substance, comprising:
an optical fiber for receiving and returning an optical signal for transducing the measured temperature and relative humidity;
an optical cavity for humidity measurement comprising a hydrophilic polymer layer deposited onto a tip of the optical fiber forming a Fabry-Perot interference, FPI;
a Fiber Bragg Grating, FBG, inscribed in the optical fiber for temperature measurement; and
a chamber comprising the tip of the optical fiber;
wherein said chamber is enclosed by a vapor-permeable, liquid-impermeable sheet.

2. The device according to the previous claim, comprising a metal oxide layer between the hydrophilic polymer layer and the tip of the optical fiber.

3. The device according to claim 2, wherein the metal oxide layer is a Titanium Dioxide, TiO₂, layer.

4. The device according to any of the previous claims, wherein the vapor-permeable, liquid-impermeable sheet is a membrane, in particular a Polydimethylsiloxane, PDMS, membrane.

5. The device according to any of the previous claims, wherein the hydrophilic polymer layer is selected from a group consisting of polyvinylpyrrolidone, PVP, polyimide, hydroxyethyl cellulose, and polyethylene glycol.

6. The device according to any of the previous claims, wherein the optical fiber comprises an FBG optical fiber section comprising said FBG, spliced to an FPI optical fiber section comprising said FPI.

7. The device according to any of the previous claims, wherein the optical fiber is coupled to a fiber optic connector.

8. The device according to any of the previous claims, comprising a ceramic ferrule wherein the tip of the optical fiber is inserted into the ceramic ferrule to provide a base surface for deposition of the hydrophilic polymer layer.

9. The device according to any of the previous claims, wherein the optical fiber is a single-mode fiber operating in the infrared region, preferably between 1500 nm and 1600 nm.

10. The device according to any of the previous claims, comprising a signal processor configured for determining relative humidity by measuring a change in an interference pattern of the reflected optical signal, in particular wherein the signal processor is configured for quantifying said change by measuring spectral separation between consecutive interference peaks or a mean spectral distance between such peaks.

11. The device according to claim 10, wherein analysis of the reflected optical signal excludes a FBG reflection peak.

12. The device according to any of the previous claims, comprising a protective encapsulation comprising a ferrule housing, stainless steel tube, and/or resin encapsulation.

13. Use of the device according to any of the previous claims for temperature and relative humidity dual-measurement of a cementitious material or substance, or temperature and relative humidity dual-measurement of an agricultural, food-tech, pharmaceutical or pyrotechnical material.

14. A method of fabricating the optical sensing device according to any of the claims 1-12, comprising:
applying a titanium dioxide, TiO₂, layer over the tip of the optical fiber or other high refractive index material;
preparing a Fabry-Perot cavity on a ceramic ferrule by dip-coating the tip of the optical fiber in a hydrophilic polymer;
incorporating a Fiber Bragg Grating sensor for temperature measurement; and assembling a protective housing comprising a ferrule and an outer casing.

15. A method of calibrating the optical sensing device according to any of the claims 1-12, comprising:
exposing the relative humidity sensor to controlled humidity environments and recording reflected spectra for relative humidity calibration;
establishing a calibration curve correlating relative humidity values to free spectral range, FSR, measurements; and
correlating Bragg wavelength shifts to known temperature values for temperature calibration.
